# EUROPÄISCHE PATENTSCHRIFT

(11) **EP 3 447 471 B1**
(45) Veröffentlichungstag und Bekanntmachung des Hinweises auf die Patenterteilung: **08.07.2020**
(21) Anmeldenummer: 17001710.7
(22) Anmeldetag: 17.10.2017
(51) Int. Cl.: G01N 15/14, G01N 21/53, A61M 1/28, G01N 15/00, G01N 15/10, G01N 15/06, G01N 21/51

(54) **VORRICHTUNG ZUR ERFASSUNG VON LEUKOZYTEN IN EINER WÄSSRIGEN SPÜLLÖSUNG**
DEVICE FOR DETECTING LEUKOCYTES IN AN AQUEOUS RINSING SOLUTION
DISPOSITIF DE DÉTECTION DE LEUCOCYTES DANS UNE SOLUTION POUR IRRIGATION AQUEUSE

(30) Priorität: 25.08.2017 DE 102017008012
(43) Veröffentlichungstag der Anmeldung: 27.02.2019
(73) Patentinhaber: Becker, Franz Ferdinand, 63110 Rodgau (DE); Hinkel, Ulrich Paul, 99425 Weimar (DE)
(72) Erfinder: Becker, Franz Ferdinand, 63110 Rodgau (DE); Hinkel, Ulrich Paul, 99425 Weimar (DE)
(74) Vertreter: Flosdorff, Jürgen

(56) Entgegenhaltungen:
- EP-A1- 0 491 971
- JP-B2- 3 549 262
- US-A1- 2008 098 798
- US-A1- 2009 078 047
- US-A1- 2017 136 166

## Beschreibung

Die Erfindung betrifft eine Apparatur, die in Flüssigkeiten nach deren medizinischer Anwendung eine durch Leukozyten und/oder Lumineszenz im PD (Peritonealdialyse) -Auslaufbeutel oder im Schlauchsystem verursachte Trübung erfasst. Die Trübung durch Leukozyten und oder deren Lumineszenz kann durch Bakterien, Zellen oder sonstige Partikel verursacht werden. Bei den Flüssigkeiten handelt es sich bevorzugt um Flüssigkeiten für den Einsatz bei allen PD-Lösungen, die bei den verschiedenen Peritonealdialyse-Verfahren eingesetzt werden, oder um Spüllösungen, die bei der Spülung von Organen und des Peritoneums eingesetzt werden. Die Erfindung ist nicht auf die Erfassung und Messung der Trübungen und Leukozyten der vorstehend aufgeführten Spüllösungen beschränkt, sondern ganz allgemein auf Flüssigkeiten verschiedenster Art anwendbar.

Außerdem kann die Vorrichtung bzw. Apparatur auch andere veränderte Eigenschaften der Flüssigkeit anzeigen, die durch Bakterien, Zellen oder Partikel verursacht werden.

Die US 2017/0136166 A1 befasst sich mit einer Vorrichtung zur Erfassung von Trübungen in bei der Peritonealdialyse verwendeten Flüssigkeiten. Die Druckschrift beschreibt ein Standgerät, bei dem die zu untersuchende Flüssigkeit in einen Behälter eingefüllt werden muss, der in das Standgerät eingesetzt wird. Das Standgerät enthält eine Lichtquelle, die den Behälter durchleuchtet, eine Detektoreinrichtung, auf die die Lichtquelle gerichtet ist, und eine Anzeigeeinrichtung für die der erfassten Trübung entsprechenden Werte. Von der zu überwachenden Flüssigkeit wird eine Probe in den Behälter eingefüllt, der dann in das Standgerät zur Untersuchung der Flüssigkeit eingesetzt wird. Diese Vorgehensweise ist mit einem beträchtlichen Aufwand verbunden und nur dazu geeignet, von Zeit zu Zeit Ergebnisse über den Zustand der z.B. bei einer Peritonealdialyse anfallenden Flüssigkeiten zu liefern.

Die EP 0 491 971 A1 offenbart eine Anordnung zur Überwachung von mittels Drainageschläuchen abgeleiteten Körperflüssigkeiten, wobei es bei der Überwachung darum geht, Blut in der Körperflüssigkeit infolge einer postoperativen Nachblutung zu erfassen. Die dabei verwendete Vorrichtung hat die Form einer Klammer, die einen Drainageschlauch zwischen den Klammerhälften einklemmt. Eine Klammerhälfte ist mit einer Lichtquelle versehen, deren Licht auf eine Detektoreinrichtung an dem anderen Klammerteil gerichtet ist. Die beiden Klammerteile haben keine Einbuchtungen, so dass die Lage eines zu durchleuchtenden Schlauchs nicht genau definiert ist, was sich negativ auf die Genauigkeit der ermittelten Werte auswirken kann. Die Detektoreinrichtung übermittelt die erfassten Werte an eine getrennt von dem Klammerteil angeordnete Auswertungsschaltung.

Die US 2008/0098798 A1 befasst sich mit einem Detektor, der Luftblasen in einer Flüssigkeit erfasst, die einen Schlauch durchströmt. Der Luftblasendetektor hat die Form einer Klammer, an deren Klammerhälften Klemmstücke gelenkig befestigt sind, in denen Sensoren untergebracht sind, um in einem zwischen den Klemmstücken festgeklemmten Schlauch eventuelle Luftblasen zu erfassen.

Es besteht seit langem ein Bedarf nach einer Vorrichtung, die bevorzugt im On-Line-Verfahren Veränderungen der Eigenschaft in Flüssigkeiten feststellen kann, nachdem die Flüssigkeiten bevorzugt zu medizinischer Anwendung oder auch Anwendung nicht-medizinischer Art benutzt wurde.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die erfindungsgemäße Vorrichtung enthält einen Basiskörper, wenigstens eine Lichtquelle, eine Detektoreinrichtung, auf die wenigstens eine Lichtquelle gerichtet ist, eine Halterung für ein die Spüllösung enthaltendes Bauteil zwischen der wenigstens einen Lichtquelle und der Detektoreinrichtung und eine Auswerte- und Anzeigeeinrichtung für die der Leukozytenmessung entsprechenden Werte. In der Vorrichtung bzw. Apparatur wird ein durchsichtiges, die Flüssigkeit enthaltendes oder die fließende Flüssigkeit führendes Bauteil durchstrahlt und die nicht absorbierte Lichtmenge oder die durch Lumineszenz entstehende Lichtmenge mit Hilfe der Detektoreinrichtung erfasst. Die erfasste Lichtmenge gibt Aufschluss über die erfassten Leukozyten oder Lumineszenz der Flüssigkeit. Die erfasste Lichtmenge wird durch eine Auswerteeinrichtung analysiert, und die so ermittelten Messwerte werden durch eine Anzeigeeinrichtung bevorzugt optisch angezeigt. Die ermittelten Messwerte können auch akustisch oder durch Vibration angezeigt werden.

Die Detektoreinrichtung kann hierzu einen Sensor zur Messung der nicht absorbierten Lichtmenge und/oder der durch Lumineszenz entstehenden Lichtmenge aufweisen. Ebenso kann die Detektoreinrichtung einen Sensor enthalten, der Bakterien, Zellen oder Partikel durch Zugabe eines Indikators zu der Flüssigkeit identifiziert und misst. Diese Verunreinigungen können durch einen geeigneten Sensor auch durch Färbung der Flüssigkeit identifiziert und gemessen werden. Mit einem Mikroskop können ebenfalls die Veränderungen der Flüssigkeit erfasst werden. Eine weitere Möglichkeit besteht darin, dass die Detektoreinrichtung Sensoren hat, die eine veränderte elektrische Leitfähigkeit, eine veränderte Viskosität, Dichte oder Oberflächenspannung der Flüssigkeit erfasst bzw. misst.

Die Vorrichtung kann auch mit einem akustischen Signalgeber versehen sein, um einen Hinweis auf die gemessenen Leukozyten oder deren Lumineszenz zu geben. Es kann auch ein bestimmter Grad der Veränderung der Eigenschaften der Flüssigkeiten festgelegt werden, bei dessen Erreichung eine Alarmierung erfolgen soll.

Die Vorrichtung ist im stationären Bereich, ambulanten Bereich und im Pflegebereich einsetzbar. Sie ist auch im häuslichen Bereich von einem Patienten selbständig anwendbar.

Der Basiskörper der Vorrichtung hat die Form einer Klammer, die aus zwei Klammerteilen besteht, die gelenkig miteinander verbunden sind und durch eine Feder in eine geschlossene Lage beaufschlagt werden. Die zwei Klammerteile haben zwei gegenüber liegende Einbuchtungen, in denen ein durchsichtiger Schlauch, eine Kanüle oder ein anderes Behältnis für die Spüllösung fixierbar ist. Im Falle des Schlauches kann die Messung einer durchfließenden Flüssigkeit erfolgen, während im Fall einer festgeklemmten Kanüle die Messung an ruhender Flüssigkeit erfolgt.

Die Halterung für das Behältnis der Spüllösung kann, in einer nicht zur Erfindung gehörende Vorrichtung, auch aus nur einer Einbuchtung in einem Klammerteil bestehen, während das andere Klammerteil das Behältnis festklemmt, so dass es in der Klammer fixiert ist.

An einem Klammerteil sind die wenigstens eine Lichtquelle und ein Gehäuse mit einem Stromanschluss und an dem anderen Klammerteil sind die Detektoreinrichtung und die Auswerte- und Anzeigeeinrichtung befestigt. An einem Klammerteil kann eine Klemme befestigt sein, so dass die gesamte Vorrichtung z.B. an einer Infusionsstange festklemmbar ist.

Bei einer anderen nicht zur Erfindung gehörenden Ausführungsform der Vorrichtung ist der Basiskörper ein bevorzugt auf Füßen stehendes Standgehäuse mit einer Einbuchtung in der Oberseite, in die ein durchsichtiges Behältnis für die Spüllösung einsetzbar ist, wobei die wenigstens eine Lichtquelle und die Detektoreinrichtung einander gegenüber liegend am Rand der Einbuchtung angeordnet sind, so dass das Licht den unteren Bereich des Behältnisses für die Spüllösung durchstrahlt.

Das Behältnis kann ein starrer Trichter sein, in den ein ebenfalls durchsichtiger, flexibler Beutel mit der Spüllösung einsetzbar ist. In diesem Fall erfolgt die Messung an der ruhenden Flüssigkeit.

Die Stromversorgung für die wenigstens eine Lichtquelle, die Detektoreinrichtung, die Auswerteeinrichtung und die Anzeigeeinrichtung ist bevorzugt in dem Standgehäuse untergebracht.

Als Lichtquelle wird bevorzugt ein Laser eingesetzt, wobei auch mehrere Laser zur Anwendung kommen können.

Weitere Einzelheiten der Erfindung sind den beigefügten Zeichnungen und der zugehörigen Beschreibung entnehmbar. Dabei zeigen auf rein schematische Weise:
- Figuren 1A und 1B: eine erste Ausführungsform der erfindungsgemäßen Vorrichtung in einer Seitenansicht und einer Aufsicht;
- Figuren 2A und 2B: eine nicht zur Erfindung gehörende Vorrichtung, ebenfalls in einer Seitenansicht und einer Aufsicht.

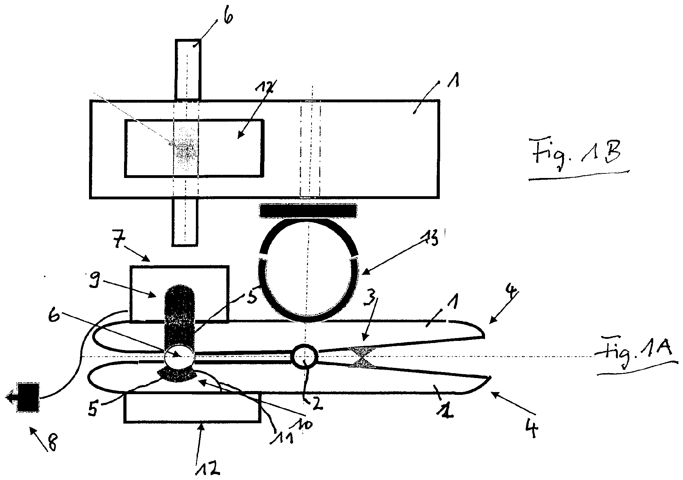

Die Figuren 1A und 1B zeigen eine Ausführungsform in der Art eines Klammer-Systems. Der Basiskörper der Vorrichtung hat die Form einer Klammer, die aus zwei Klammerteilen 1 zusammengesetzt ist. Die Klammerteile 1 sind etwa in der Mitte ihrer Längserstreckung durch ein Gelenk 2 miteinander verbunden. Eine zwischen den Klammerteilen 1 angeordnete Feder 3 drückt die Enden der beiden Klammerteile 1 auseinander in die geschlossene Stellung der Klammer. Die Klammer ist dadurch zu öffnen, dass auf die Enden 4 der beiden Klammerteile 1 durch Finger des Benutzers Druck ausgeübt wird. Im vorderen Bereich der Klammer sind in den beiden Klammerteilen 1 einander gegenüber liegende Einbuchtungen 5 ausgebildet, in denen ein Schlauch 6 fixierbar ist, durch den die zu untersuchende Flüssigkeit strömt. Auf dieselbe Weise kann z.B. auch eine Kanüle mit einer ruhenden Flüssigkeit in der Klammer gehalten sein.

An dem in der Figur 1A oberen Klammerteil 1 ist ein Gehäuse 7 befestigt, in dem ein Stromanschluss angeordnet ist, der von einem externen Ladegerät 8 mit Netzspannung oder von einem Akku mit Strom versorgt wird. In dem Gehäuse 7 ist außerdem eine Lichtquelle 9 angeordnet, deren Licht durch einen Kanal in dem oberen Klammerteil 1 zu dem Schlauch 6 verläuft. In dem anderen Klammerteil ist auf der gegenüber liegenden Seite ein Detektor 10 untergebracht, der mit einer Auswerteeinrichtung versehen ist und über eine Leitung 11 mit einem Anzeigefeld 12 verbunden ist, das auf der Außenseite der in der Figur unteren Klammer 1 befestigt ist.

An dem oberen Klammerteil 1 ist eine Klemme 13 angebracht, mit der die gesamte Vorrichtung z.B. an einer Infusionsstange befestigbar ist.

Die Figur 1B zeigt in einer Seitenansicht das Klammerteil 1 mit dem Anzeigefeld 12 und ein schlauchförmiges Behältnis 6 für die zu untersuchende Flüssigkeit.

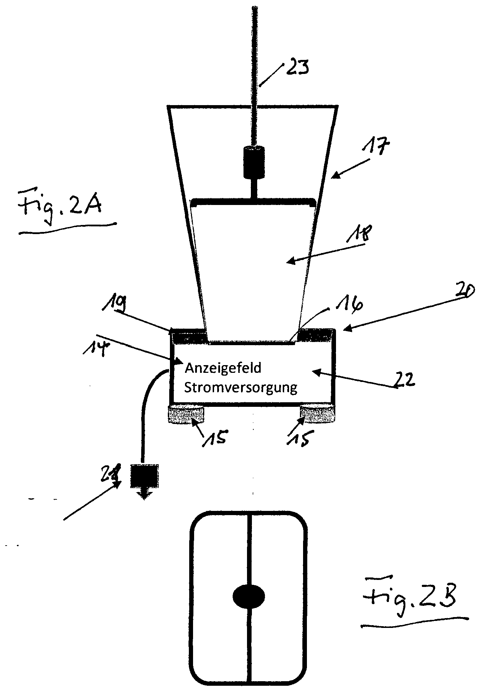

Die Figuren 2A und 2B zeigen eine nicht zur Erfindung gehörende Vorrichtung. Ein Standgehäuse 14, das auf Füßen 15 ruht, hat in seiner Oberseite eine Einbuchtung 16. In diese Einbuchtung 16 ist ein starrer Trichter 17 eingesetzt, der einen flexiblen Beutel 18 aufnehmen kann. Dieser flexible Beutel wird über eine Leitung mit der benutzten Spüllösung gefüllt. Der flexible Beutel kann auch nach seiner Befüllung in den Trichter 17 zur Messung eingesetzt werden. In dem Standgehäuse sind zu beiden Seiten der Einbuchtung 16 eine Lichtquelle 19 und eine Detektor-einrichtung 20 angeordnet, die einander gegenüber liegen. Das ausgesandte Licht durchstrahlt dabei den unteren Bereich des Trichters 17 und des flexiblen Beutels mit der Flüssigkeit.

In dem Standgehäuse 14 ist außerdem die Stromversorgung für die Lichtquelle 19, die Detektoreinrichtung 20 und eine Auswerteeinrichtung für die ermittelten Werte untergebracht. Die Stromversorgung erfolgt durch Akkubetrieb oder Netzbetrieb 21. An einer Außenseite des Standgehäuses 14 ist ein Anzeigefeld 22 für die ermittelten Werte angebracht.

Der flexible Beutel 18 ist an seiner Oberseite mit einem flexiblen Schlauch 23 versehen über den der flexible Beutel 18 mit der genutzten Spüllösung gefüllt wird und aus dem Trichter 17 herausgezogen werden kann.

## Patentansprüche

1. Vorrichtung zur Erfassung von Leukozyten und sonstigen Trübungen in einer wässrigen Spüllösung nach deren medizinischer oder nicht-medizinischer Anwendung,
mit einem Basiskörper, wenigstens einer Lichtquelle (9), einer Detektoreinrichtung (10), auf die die wenigstens eine Lichtquelle gerichtet ist, einer Halterung für ein die Spüllösung enthaltendes Bauteil (6) zwischen der wenigstens einen Lichtquelle und der Detektoreinrichtung und einer Anzeigeeinrichtung (12) für die der erfassten Leukozyten und sonstigen Trübungen entsprechenden Werte,
wobei der Basiskörper die Form einer Klammer hat, die aus zwei Klammerteilen (1) besteht, die durch ein Gelenk (2) miteinander verbunden und durch eine zwischen den Klammerteilen (1) angeordnete Feder (3) in eine geschlossene Lage beaufschlagt sind,
dass die Halterung zwei gegenüber liegende Einbuchtungen (5) in den Klammerteilen (1) aufweist, in denen ein durchsichtiger Schlauch (6) oder eine Kanüle für die Spüllösung fixierbar ist,
dass die Klammer dadurch zu öffnen ist, dass auf die Enden der Klammerteile (1) Druck ausgeübt wird, und
dass an einem Klammerteil (1) die wenigstens eine Lichtquelle (9) und ein Gehäuse (7) mit einem Stromanschluss und an dem anderen Klammerteil (1) die Detektoreinrichtung (10) und die Anzeigeeinrichtung (12) befestigt sind.

2. Vorrichtung nach Anspruch 1,
**dadurch gekennzeichnet,**
**dass** an einem Klammerteil (1) eine Klemme (13) befestigt ist, mit der die Vorrichtung an einer Infusionsstange festklemmbar ist.

3. Vorrichtung nach einem der Ansprüche 1 oder 2,
**dadurch gekennzeichnet,**
**dass** die Detektoreinrichtung einen Sensor zur Messung der nicht absorbierten Lichtmenge und/oder der durch Lumineszenz entstehenden Lichtmenge, und/oder einen Sensor zur Messung der elektrischen Leitfähigkeit und/oder einen Sensor zur Messung der Viskosität, der Dichte oder Oberflächenspannung aufweist.

4. Vorrichtung nach einem der Ansprüche 1 bis 3,
**dadurch gekennzeichnet,**
**dass** als Lichtquelle (9) ein oder mehrere Laser verwendet wird/werden.

## Claims

1. Apparatus for detecting leukocytes and other cloudy materials in an aqueous rinsing solution after its medical or non-medical use with a base body, at least one light source (9), a detector device (10), towards which the at least one light source is directed, a mounting for a component (6), which contains the rinsing solution, between the at least one light source and the detector device and an indicator device (12) for the values corresponding to the detected leukocytes and other cloudy materials, wherein the base body has the form of a clip, which consists of two clip portions (1), which are connected together by a joint (2) and are urged into a closed position by a spring (3) arranged between the clip portions (1), that the mounting has two opposing indentations (5) in the clip portions (1), into which a transparent hose (6) or a cannula for the rinsing solution may be fixed, that the clip may be opened by exerting pressure on the ends of the clip portions (1), and that fastened to one clip portion (1) are the at least one light source and a housing (7) with an electrical power connection and to the other clip portion (1) are the detector device (10) and the indicator device (12).

2. Apparatus as claimed in Claim 1, **characterised in that** secured to one clip portion (1) there is a clamp (13), with which the apparatus may be clamped to an infusion pole.

3. Apparatus as claimed in one of Claims 1 or 2, **characterised in that** the detector device includes a sensor for measuring the amount of light which is not absorbed and/or the amount of light produced by luminescence and/or a sensor for measuring the electrical conductivity and/or a sensor for measuring the viscosity, the density or surface tension.

4. Apparatus as claimed in one of Claims 1 to 3, **characterised in that** one or more lasers is/are used as the light source (9).

## Revendications

1. Dispositif de détection de leucocytes et d'autres turbidités dans une solution de rinçage aqueuse après son utilisation médicale ou non médicale,
avec un corps de base, au moins une source de lumière (9), un système détecteur (10), sur lequel l'au moins une source de lumière est dirigée, un support pour un composant (6) contenant la solution de rinçage entre l'au moins une source de lumière et le système détecteur et un système d'affichage (12) pour les valeurs correspondant aux leucocytes et autres turbidités détectés,
dans lequel
le corps de base a la forme d'une attache, qui est constituée de deux parties d'attache (1), qui sont reliées l'une à l'autre par une articulation (2) et sont soumises à l'action d'un ressort (3) disposé entre les parties d'attache (1) dans une position fermée,
dans lequel le support présente deux indentations (5) se faisant face dans les parties d'attache (1), dans lesquelles un tuyau flexible (6) transparent ou une canule pour la solution de rinçage peut être fixé(e),
dans lequel l'attache est à ouvrir en ce qu'une pression est exercée sur les extrémités des parties d'attache (1), et
dans lequel l'au moins une source de lumière (9) et un boîtier (7) avec un raccordement électrique sont fixés au niveau d'une partie d'attache (1) et le système détecteur (10) et le système d'affichage (12) sont fixés au niveau de l'autre partie d'attache (1).

2. Dispositif selon la revendication 1,
**caractérisé en ce**
**qu'**est fixée au niveau d'une partie d'attache (1), une pince (13), par laquelle le dispositif peut être immobilisé par serrage au niveau d'une tige de perfusion.

3. Dispositif selon l'une quelconque des revendications 1 ou 2,
**caractérisé en ce**
**que** le système détecteur présente un capteur pour mesurer la quantité de lumière non absorbée et/ou la quantité de lumière apparaissant par luminescence et/ou un capteur pour mesurer la conductivité électrique et/ou un capteur pour mesurer la viscosité, la densité ou la tension de surface.

4. Dispositif selon l'une quelconque des revendications 1 à 3,
**caractérisé en ce**
**qu'**un ou plusieurs lasers sont utilisés en tant que source de lumière (9).
